# FASCICULE DE BREVET EUROPEEN

(11) **EP 1 525 876 B1**
(45) Date de publication et mention de la délivrance du brevet: **22.05.2013**
(21) Numéro de dépôt: 04300702.0
(22) Date de dépôt: 21.10.2004
(51) Int. Cl.: A61K 8/81, A61Q 1/06, A61Q 1/10, A61K 8/02

(54) **Composition cosmétique thermogonflante**
Thermoquellbare kosmetische Zusammensetzung
Thermo-swellable cosmetic composition

(30) Priorité: 24.10.2003 FR 0312503
(43) Date de publication de la demande: 27.04.2005
(73) Titulaire: L'Oréal, 75008 Paris (FR)
(72) Inventeur: de la Poterie, Valérie, 77820 Le Chatelet-en-Brie (FR); Daubige, Thérèse, 77480 Mousseaux les Bray (FR)
(74) Mandataire: Le Coupanec, Pascale A.M.P.

(56) Documents cités:
- EP-A- 0 770 373
- EP-A- 1 132 076
- WO-A-03/028679
- US-A- 3 615 972
- US-A- 4 964 429
- US-A- 5 853 010
- US-A- 6 009 884

## Description

La présente invention concerne des compositions cosmétiques comprenant au moins un composé susceptible de gonfler à la chaleur, ces compositions étant capables de conférer du volume lorsqu'elles sont soumises à une source de chaleur.

« Conférer du volume », encore ci-après qualifié d'effet volumateur, est un effet fréquemment recherché dans le domaine cosmétique.

Dans le domaine du maquillage des cils, il a par exemple été proposé des formulations de mascara très concentrées, à extrait sec élevé, permettant de déposer une forte épaisseur de matière. Cependant, de telles formulations présentent l'inconvénient d'être très consistantes et donc difficilement applicables. Toujours dans le domaine des mascaras il a, à l'inverse, été proposé des formulations très fluides qui par superposition de couches permettent également l'obtention d'un effet volumateur. Toutefois là encore, les conditions d'obtention de l'effet volumateur ne sont pas totalement satisfaisantes car leur mise en oeuvre est délicate et longue.

Pour les lèvres, l'effet volumateur a jusqu'à présent été obtenu soit en dessinant un contour des lèvres plus large que le contour naturel, soit en jouant sur des effets optiques par exemple à l'aide de compositions brillantes.

Une autre alternative a consisté à faire usage, dans des compositions cosmétiques, de polymères dotés d'une forte capacité d'absorption de l'eau. La mise en contact de la composition, appliquée sur son site de maquillage, avec de l'eau, provoque une augmentation de son volume initial, générant ainsi l'effet volumateur recherché. De telles compositions sont notamment décrites dans les documents US 6 045 783 et EP 1 228 747. Les compositions à effet volumateur utilisant ces polymères sont cependant peu satisfaisantes car l'obtention d'un gonflement significatif nécessite l'apport d'une quantité d'eau assez importante. De plus, ce gonflement n'est pas durable et est réversible avec l'évaporation de l'eau.

Il est connu de WO 03/028679 des compositions de coiffage comprenant un polymère adhésif de masse moléculaire moyenne en poids supérieure à 20000, de 0,01 à 20 % en poids de particules et un véhicule aqueux, ainsi que leur utilisation pour améliorer le volume d'une coiffure *via* le dépôt desdites particules sur les cheveux.

Le document EP 1 132 076 décrit, pour sa part, des compositions capillairescomprenant un agent épaississant, un polymère fixant et un composé pulvérulent, et leur mise en oeuvre pour fixer et/ou mettre en forme une coiffure.

On a maintenant découvert qu'il était possible d'obtenir des compositions cosmétiques capables de générer un effet volumateur particulièrement avantageux et ceci d'une manière prolongée dans le temps.

Selon un premier aspect, la présente invention concerne une composition cosmétique de maquillage, en particulier thermoexpansible, choisie parmi un mascara, un eyeliner et un rouge à lèvres comprenant au moins des particules capables de s'expanser sous l'action d'une température supérieure ou égale à 45 °C.

Par l'expression « composition thermoexpansible », on désigne une composition susceptible de s'expanser, c'est-à-dire d'augmenter son volume à la chaleur.

Selon un autre aspect, la présente invention concerne un procédé de maquillage, comprenant le fait d'appliquer une composition telle que définie précédemment sur un support à traiter, et comprenant en outre le fait de chauffer ladite composition, en particulier pendant ou postérieurement à ladite application, dans des conditions permettant l'augmentation du volume de ladite composition par rapport à son volume non chauffé.

Selon un autre aspect, la présente invention a pour objet un support maquillé constitué d'un accessoire de maquillage comprenant un maquillage susceptible d'être obtenu selon le procédé tel que défini précédemment.

Selon un autre aspect, la présente invention a également pour objet un ensemble de conditionnement et d'application d'une composition comprenant au moins des particules capables de s'expanser sous l'action d'une température supérieure ou égale à 45 °C, notamment d'une composition de maquillage et/ou de soin, notamment des cils ou des sourcils, telle que définie précédemment, comprenant :
i) un réservoir comprenant ladite composition,
ii) un dispositif pour l'application de la composition ; et
iii) des moyens de chauffage.

Selon un autre aspect, la présente invention concerne l'utilisation de particules thermoexpansibles telles que définies précédemment à titre d'additif pour conférer un effet volumateur sous l'effet de la chaleur dans une composition cosmétique de maquillage choisie parmi un mascara, un eyeliner et un rouge à lèvres.

Comme il ressort des exemples ci-après, les compositions selon l'invention manifestent un effet volumateur significatif. En particulier, le volume initial de la composition, c'est-à-dire celui obtenu à l'issue de son application sur le support à traiter peut être augmenté, en réponse à une source de chaleur, d'un facteur multiplicatif d'au moins 1,5, notamment de 2, en particulier de 2,5, plus particulièrement de 3 et voire jusqu'à 10. Cet effet volumateur peut notamment être proportionnel à la teneur en particules capables de s'expanser sous l'action d'une température supérieure ou égale à 45 °C.

Cet effet volumateur peut être apprécié par mesure de l'épaisseur de composition appliquée sur le support à traiter, avant chauffage et après chauffage. Dans le cas d'une composition de maquillage pour les cils, cet effet peut être caractérisé par mesure du diamètre de la fibre maquillée.

Cet effet volumateur est obtenu en quelques secondes et est durable dans le temps.

### Composé susceptible de gonfler à la chaleur

Comme indiqué précédemment, les compositions selon l'invention comprennent au moins des particules capables de s'expanser sous l'action d'une température supérieure ou égale à 45 °C.

Il peut notamment s'agir d'un composé qui réagit, sous l'action de la chaleur, pour libérer un gaz qui se trouve emprisonné dans la matrice du dépôt.

Il s'agit de particules thermoexpansibles.

Par l'expression « particules thermoexpansibles », on désigne plus particulièrement des particules susceptibles de se déformer et de s'expanser à la chaleur. Les particules au sens de la présente invention peuvent encore être des particules thermodéformables non expansées. Elles se distinguent à ce titre des particules expansées qui ne sont précisément plus sujettes à une déformation sous l'action de la chaleur à l'image par exemple des particules de chlorure de polyvinylidène/acrylonitrile commercialisées sous la dénomination générale d'« Expancel^{®} » par la société AKZO NOBEL sous les références particulières « Expancel^{®} WE » ou « DE ».

Les particules utilisées dans les compositions selon l'invention sont capables de s'expanser sous l'action d'une température généralement supérieure ou égale à 45 °C, notamment supérieure ou égale à 50 °C, en particulier supérieure ou égale à 60 °C, plus particulièrement supérieure ou égale à 70°C. En particulier, il peut s'agir d'une température supérieure ou égale à 80 °C, notamment supérieure ou égale à 85 °C, en particulier supérieure ou égale à 90 °C, plus particulièrement supérieure ou égale à 100 °C, et allant jusqu'à 190-200 °C.

Avantageusement, ces particules ne sont pas sensibles à la présence d'eau.

Avantageusement, la déformation et l'expansion desdites particules sous l'effet de la chaleur sont irréversibles.

En particulier, les particules utilisées dans la présente invention sont thermoplastiques. Par l'expression « thermoplastique », on désigne des particules qui sont susceptibles de se déformer sous l'action de la chaleur et de conserver leur nouvelle forme y compris après refroidissement notamment à température ambiante (25°C).

Les particules utilisées dans la présente invention sont généralement des particules creuses comportant une enveloppe continue et au moins une cavité.

L'enveloppe des particules est flexible pour se prêter à une déformation mécanique. Elle comprend généralement au moins un polymère, homo- ou co-polymère, formé à partir de monomères à insaturations éthyléniques. Des exemples de telles particules sont notamment décrits dans les documents EP-A-56219, EP-A-348 372, EP-A-486 080, EP-A-320 473, EP-A-112 807 et US-A-3 615 972.

Les monomères utilisés peuvent être en particulier des esters d'acide méthacrylique ou acrylique, tels que l'acrylate et le méthacrylate de méthyle, le chlorure de vinylidène, l'acrylonitrile, le styrène et ses dérivés, le butadiène et ses dérivés, et leurs mélanges.

A titre représentatif et non limitatif des polymères susceptibles de composer l'enveloppe des particules utilisées dans la présente invention, on peut notamment citer des polymères comportant au moins des motifs dérivés d'acrylate ou de méthacrylate de méthyle, des polymères comportant au moins des motifs dérivés d'acrylonitrile, des polymères comportant au moins des motifs dérivés d'acrylonitrile et de méthacrylate de méthyle, des polymères comportant au moins des motifs dérivés de styrène et d'acrylonitrile, des polymères comportant au moins des motifs dérivés de chlorure de vinylidène et d'acrylonitrile et des polymères comportant au moins des motifs dérivés de chlorure de vinylidène et de chlorure de vinyle. En particulier, ledit polymère peut être choisi parmi les polymères de chlorure de vinylidène/acrylonitrile/méthacrylate de méthyle, les polymères d'acrylonitrile/méthacrylate de méthyle et les homopolymères d'acrylonitrile.

Les particules contiennent généralement au sein d'une ou plusieurs cavité(s) au moins un composé capable de manifester, en réponse à un chauffage à une température située dans une gamme allant de 45 °C à 200 °C et à pression sensiblement constante, une augmentation significative de son volume par rapport à son volume à température ambiante.

On entend par l'expression « augmentation significative de son volume », une augmentation d'au moins un facteur 30, en particulier d'au moins un facteur 40 et plus particulièrement d'au moins un facteur 50 du volume occupé.

De façon générale, le composé contenu à l'intérieur de la cavité peut être à température ambiante un composé gazeux ou bien un composé liquide présentant une température de vaporisation située dans la gamme de 45 °C à 200 °C, en particulier dans la gamme 80 °C à 200 °C, et plus particulièrement supérieure ou égale à 100 °C.

Selon une première variante, ce composé est à l'état gazeux dans la particule et se dilate sous l'effet de la chaleur. Parmi les composés à l'état gazeux à température ambiante et pression atmosphérique (10⁵ Pa), on peut citer l'air, l'azote, les hydrocarbures tels qu'en particulier ceux comprenant 1, 2, 3 ou 4 atomes de carbone comme par exemple le butane, l'isobutane et leurs mélanges.

Selon une deuxième variante, le composé contenu dans la cavité des particules est un composé liquide à température ambiante et pression atmosphérique tel que défini précédemment. Parmi ces composés, on peut citer les hydrocarbures, notamment ayant de 5 à 15, en particulier de 5 à 12 et plus particulièrement de 5 à 10 atomes de carbone. Il peut s'agir en particulier d'un composé choisi parmi le n-pentane, l'iso-pentane et le néopentane.

La température d'expansion de la particule dépend à la fois de la nature du composé présent dans sa cavité, et de celle du polymère formant son enveloppe, et peut en particulier aller de 45 à 200 °C, et être notamment supérieure ou égale à 70°C, en particulier à 80 °C, et plus particulièrement supérieure ou égale à 100 °C.

Les particules utilisées dans les compositions selon l'invention peuvent être sèches ou hydratées.

Ces particules peuvent être de différentes formes. Elles peuvent être de forme globulaire, voire sphérique, ou peuvent également être allongées.

Selon un mode de réalisation particulier, les particules non expansées de l'invention sont sphériques et présentent une granulométrie, exprimée en diamètre « effectif » moyen en poids D[0,5], allant de 0,5 µm à 200 µm, notamment de 1 µm à 100 µm, en particulier de 4 µm à 50 µm et plus particulièrement de 5 µm à 40 µm.

Selon un mode de réalisation particulier, les particules utilisées dans les compositions selon l'invention ont une forme de fibre. Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150. Les fibres présentent en particulier une longueur allant de 0,05 mm à 6 mm.

Les particules non expansées utilisées dans la présente invention présentent généralement une masse volumique allant de 500 kg/m³ à 5000 kg/m³, en particulier de 900 kg/m³ à 3000 kg/m³ et plus particulièrement de 900 kg/m³ à 2000 kg/m³.

Les particules utilisées dans la présente invention peuvent être colorées ou incolores.

Comme particules utilisables dans les compositions de l'invention, on peut citer par exemple les microsphères non expansées de copolymère de chlorure de vinylidène/d'acrylonitrile/méthacrylate de méthyle comme par exemple celles commercialisées sous la dénomination d'« Expancel®» par la Société AKZO NOBEL sous les références 820 DU 40 (10-16 µm), 820 SL 40 (2-30 µm) ou 642 WU (10-16 µm), et d'acrylonitrile/méthacrylate de méthyle comme par exemple celles commercialisées sous la dénomination d'« Expancel^{®} » sous la référence 051 DU 40 (9-15 µm). Comme particules pouvant être également utilisées dans les compositions selon l'invention, on peut encore citer les microsphères non expansées d'homopolymère d'acrylonitrile comme par exemple celles commercialisées sous la dénomination d'« Expancel 007WU^{®} » (5-25 µm), de « Micropearl F-series^{®} » par la Société MATSUMOTO ou d'« Ucelite^{®} » par la Société UCB.

A titre de particules pouvant être utilisées dans les compositions selon l'invention, on peut encore citer les microsphères non expansées de polyacrylonitrile traitées en surface par de l'oxyde de titane (TiO₂), de taille moyenne 50 µm, comportant dans leur cavité un mélange de butane et de méthane, notamment de référence MFL 50STI, commercialisées par la société Matsumoto.

Les particules commercialisées sous la dénomination « Expancel^{®} » sous les références citées ci-dessus comprennent généralement dans leur cavité un composé à l'état gazeux.

Bien entendu, les particules utilisées dans la composition selon l'invention sont choisies selon le type de composition recherché et le moyen de chauffage envisagé.

Les particules thermoexpansibles telles que définies précédemment sont généralement présentes dans les compositions selon l'invention en une teneur allant de 0,05 % à 50 %, en particulier de 0,1 % à 40 %, plus particulièrement de 0,5 % à 30% en poids par rapport au poids total de la composition.

### Milieu cosmétiquement acceptable

Les compositions selon l'invention peuvent comprendre en outre au moins un milieu cosmétiquement acceptable.

Par « milieu cosmétiquement acceptable », on entend un milieu compatible avec une application sur la peau, les lèvres, les ongles et les fibres kératiniques.

Ce milieu peut être de type aqueux ou non aqueux ou comprendre une phase aqueuse et une phase non aqueuse, être non volatil ou comprendre au moins un composé volatil.

Lorsque le milieu est de type aqueux, il peut être constitué uniquement d'eau ou bien encore d'un mélange d'eau avec au moins un solvant organique.

Ces solvants organiques sont généralement hydrosolubles.

Parmi ceux-ci, on peut citer en particulier, les monoalcools inférieurs ayant de 1 à 5 atomes de carbone tels que l'éthanol, l'isopropanol, les glycols ayant de 2 à 8 atomes de carbone tel que la glycérine, le propylène glycol, l'éthylène glycol, le 1,3-butylène glycol, le dipropylène glycol, les cétones en C₃-C₄, les aldéhydes en C₂-C₄.

Lorsque le milieu est de type non aqueux, il comprend moins de 20 % en poids d'eau et/ou de solvant hydrosoluble. Il comprend des solvants organiques non solubles dans l'eau telles que des huiles.

Par « milieu ou composé volatil », on entend, au sens de l'invention, tout milieu ou composé susceptible de s'évaporer au contact de la peau, des lèvres, des ongles ou des fibres kératiniques en moins d'une heure, à température ambiante et pression atmosphérique. Le milieu ou composé volatil est liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

A l'inverse, on entend par « milieu ou composé non volatil », un milieu ou composé restant sur la peau, les lèvres, les ongles ou les fibres kératiniques à température ambiante et pression atmosphérique, au moins plusieurs heures et ayant notamment une pression de vapeur inférieure à 10⁻³ mm de Hg (0,13Pa).

Parmi les huiles volatiles, on peut citer des huiles hydrocarbonées, des huiles siliconées, des huiles fluorées ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux « d'Isopars^{®} » ou de « Permetyls^{®} », les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination « Shell Solt^{®}» par la société SHELL, peuvent aussi être utilisées.

Comme huile volatile, on peut aussi utiliser les silicones volatiles, comme par exemple les huiles de silicones linéaires ou cycliques volatiles, notamment celles ayant une viscosité ≤ 6 centistokes (6.10⁻⁶ m²/s), et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Comme composé organique volatil, on peut encore citer des solvants organiques fluorés tels que le nonafluorométhoxy butane ou le perfluorométhylcyclopentane.

Le milieu utilisé dans les compositions de l'invention peut également comprendre au moins un composé non volatil, non soluble dans l'eau et liquide à température ambiante, notamment au moins une huile non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STEARINERIES DUBOIS ou ceux vendus sous les dénominations de « Miglyol 810^{®} », « 812^{®} » et « 818^{®} » par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyldodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans la composition de l'invention sont notamment des huiles fluorosiliconées, des polyéthers fluorés, des silicones fluorées telles que décrit dans le document EP-A-847752.

Les particules sont généralement présentes en dispersion dans le milieu cosmétiquement acceptable.

La composition selon l'invention peut comprendre en outre au moins un ingrédient supplémentaire cosmétiquement acceptable.

Parmi ces ingrédients supplémentaires, on peut citer des polymères filmogènes, des matières colorantes, des charges, des épaississants ou gélifiants, des agents tensioactifs, des cires, des plastifiants, des antioxydants, des conservateurs, des parfums, des neutralisants, des actifs cosmétiques comme par exemple des émollients, des hydratants, des vitamines, des filtres solaires, et leurs mélanges.

Ainsi, selon un mode de réalisation préféré de l'invention, la composition cosmétique comprend en outre au moins une matière colorante.

Selon un autre mode de réalisation préféré, la composition selon l'invention est caractérisée en ce qu'il s'agit d'une composition cosmétique de maquillage.

Selon encore un autre mode de réalisation, la composition selon l'invention est caractérisée en ce qu'il s'agit d'une composition cosmétique de maquillage des fibres kératiniques.

### Polymère filmogène

La composition selon l'invention peut donc comprendre en outre au moins un agent filmogène.

Selon la présente invention, on entend par « polymère filmogène », un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges.

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations « Neocryl XK-90^{®} », « Neocryl A1070^{®} », « Neocryl A-1090^{®} », « Neocryl BT-62^{®} », « Neocryl A-1079^{®} », « Neocryl A-523 » par la société AVECIA-NEORESINS, « Dow Latex 432 » par la société DOW CHEMICAL, « Daitosol 5000 AD^{®}» par la société DAITO KASEY KOGYO; ou encore les dispersions aqueuses de polyuréthane vendues sous les dénominations « Neorez R-981^{®} », « Neorez R-974^{®} » par la société AVECIA-NEORESINS, les « Avalure UR-405^{®} », « Avalure UR-410^{®} », « Avalure UR-425^{®} », « Avalure UR-450^{®} », « Sancure 875^{®} », « Sancure 861^{®} », « Sancure 878^{®} », « Sancure 2060^{®} » par la société GOODRICH, « Impranil 85^{®} » par la société BAYER, « Aquamere H-1511^{®} » par la société HYDROMER ; les sulfopolyesters vendus sous le nom de marque « Eastman AQ^{®} » par la société EASTMAN CHEMICAL PRODUCTS, les dispersions vinyliques comme le « Mexomère PAM^{®} » et aussi les dispersions acryliques dans l'isododécane comme le « Mexomère PAP^{®} » de la société CHIMEX.

Lorsque la composition selon l'invention comprend au moins un polymère filmogène, celui-ci est généralement présent en une teneur en matières sèches (ou matière active) de 1 à 40 %, en particulier de 2 à 35 %, et plus particulièrement de 3 à 30 % en poids par rapport au poids total de la composition.

La composition selon l'invention peut également comprendre un agent auxiliaire de filmification favorisant la formation d'un film avec le polymère filmogène.

### Matière colorante

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ses matières colorantes peuvent être présentes en une teneur allant de 0,01 à 30 % en poids par rapport au poids total de la composition.

### Charges

La composition selon l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination « d'Orgasol^{®} » par la société ATOCHEM, de poly-β-alanine et de polyéthylène, les poudres de polymères de tétrafluoroéthylène comme le Téflon^{®} , la lauroyl-lysine, l'amidon, le nitrure de bore, les micro sphères creuses polymériques expansées telles que celles de chlorure de polyvinylidène/acrylonitrile comme celles commercialisées sous les dénominations « d'Expancel^{®} » sous les références DE ou WE par la société AKZO NOBEL, les poudres acryliques telles que celles commercialisées sous la dénomination « Polytrap^{®} » par la société DOW CORNING, les particules de polyméthacrylate de méthyle et les microbilles de résine de silicone (« Tospearls^{®} » de TOSHIBA, par exemple), le carbonate de calcium précipité, le carbonate et l'hydrocarbonate de magnésium, l'hydroxyapatite, les microsphères de silice creuses (« Silica Beads^{®} » de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

Les charges peuvent représenter de 0,1 à 25 %, en particulier de 1 à 20 % en poids par rapport au poids total de la composition.

### Gélifiant

Les gélifiants utilisables dans les compositions selon l'invention peuvent être organiques ou minéraux, polymériques ou moléculaires, lipophiles ou hydrophiles.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de « Bentone 38V^{®} » par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6^{ème} édition, 1995). Elles sont par exemple commercialisées sous les références « Aerosil R812^{®} » par la société DEGUSSA, « CAB-O-SIL TS-530^{®}» par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6^{ème} édition, 1995). Elles sont par exemple commercialisées sous les références « Aerosil R972^{®} », et « Aerosil R974^{®} » par la société DEGUSSA, « CAB-O-SIL TS-610^{®} » et « CAB-O-SIL TS-720^{®} » par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de « KSG6^{®} », « KSG16^{®} » et de « KSG18^{®} » par la société SHIN-ETSU, de « Trefil E-505C^{®} » et « Trefil E-506C^{®} » par la société DOW-CORNING, de « Gransil SR-CYC^{®} », « SR DMF10^{®} », « SR-DC556^{®} », « SR 5CYC gel^{®} », « SR DMF 10 gel^{®} » et de « SR DC 556 gel^{®} » par la société GRANT INDUSTRIES, de « SF 1204^{®}» et de « JK 113^{®} » par la société GENERAL ELECTRIC ; l'éthylcellulose comme celle vendue sous la dénomination d'« Ethocel^{®}» par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type « dibloc » ou « tribloc » du type polystyrène/polyisoprène, polystyrène/polybutadiène tels que ceux commercialisés sous la dénomination de « Luvitol HSB^{®} » par la société BASF, du type polystyrène/copoly(éthylène-propylène) tels que ceux commercialisés sous la dénomination de « Kraton^{®} » par la société SHELL CHEMICAL CO ou encore du type polystyrène/copoly(éthylène-butylène).

Parmi les gélifiants pouvant être utilisés dans les compositions selon l'invention, on peut encore citer les esters de dextrine et d'acide gras, tels que les palmitates de dextrine, notamment tels que ceux commercialisés sous les dénominations de « Rheopearl TL^{®} » ou « Rheopearl KL^{®} » par la société CHIBA FLOUR.

Ces gélifiants peuvent être présents en une teneur de 0,1 à 15%, et en particulier de 0,5 à 10 % en poids par rapport au poids total de la composition.

### Agents tensioactifs

La composition selon l'invention peut en outre contenir des agents tensioactifs émulsionnants présents notamment en une proportion allant de 0,1 à 30 % en poids, notamment de 2 à 20 % en poids, et en particulier de 5 à 15 % en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.

### Cire(s)

La composition selon l'invention peut comprendre en outre au moins une cire.

Par cire, on entend d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention peuvent être choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination « TA-TX2i^{®} » par la société RHEO, équipé d'un mobile en inox en forme de cylindre d'un diamètre de 2 mm en mesurant l'évolution de la force (force de compression ou force d'étirement) (F) en fonction du temps, pendant l'opération suivante :

Le mobile est déplacé à la vitesse de 0,1 mm/s puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté.

A titre illustratif des cires convenant à l'invention, on peut notamment citer les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale « Iso-Jojoba-50^{®} », l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de « Hest 2T-4S^{®} » par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de « Phytowax ricin 16L64^{®} » et « 22L73^{®} » par la société SOPHIM. De telles cires sont décrites dans la demande FR-A- 2792190.

Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i^{®} » par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C₂₀-C₄₀ (le groupe alkyle comprenant de 20 à 40 atomes de carbone), seul ou en mélange, en particulier un 12-(12'-hydroxystéaryloxy)stéarate d'alkyle en C₂₀-C₄₀, de formule (1) : 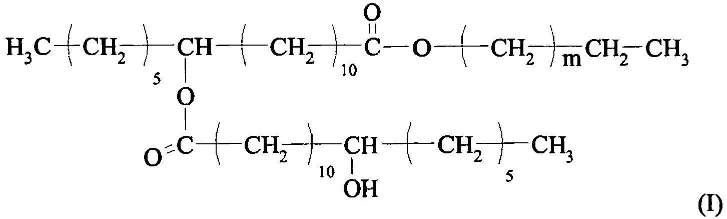 dans laquelle m est un entier allant de 18 à 38, ou un mélange de composés de formule (I).

Une telle cire est notamment vendue sous les dénominations « Kester Wax K 82 P^{®} » et « Kester Wax K 80 P^{®} » par la société KOSTER KEUNEN.

Les cires citées ci-dessus présentent généralement un point de fusion commençante inférieur à 45 °C.

La cire ou les cires peu(ven)t se présenter sous la forme d'une microdispersion aqueuse de particules de cire. On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, les homogénéisateurs haute pression, et les turbines.

Les particules de la microdispersion de cire ont en particulier des dimensions moyennes inférieures à 1 µm (notamment allant de 0,02 µm à 0,99 µm), et plus particulièrement inférieures à 0,5 µm (notamment allant de 0,06 µm à 0,5 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Dans la composition selon l'invention, on peut bien entendu utiliser une seule de ces cires ou un mélange.

Lorsque la composition selon l'invention contient au moins une cire, la teneur en cire est généralement de 1 à 60 % en poids, en particulier de 2 à 50 %, plus particulièrement de 3 à 40 % en poids de cire(s) par rapport au poids total de la composition.

Selon la nature du milieu et des éventuels ingrédients supplémentaires utilisés, la composition selon l'invention peut être de type aqueuse, hydroalcoolique, non aqueuse, voire anhydre ou bien encore sous forme d'émulsion E/H ou H/E.

Bien entendu, le choix du type de composition, du milieu et des ingrédients supplémentaires dépend de la finalité de la composition. En particulier, la composition selon l'invention peut être une composition de maquillage telle qu'un mascara, un eyeliner, ou un rouge à lèvres. Il peut également s'agir d'une composition permettant de combler les rides et ridules de la peau des lèvres pour obtenir un effet lissant.

Les compositions selon l'invention telles que définies précédemment sont généralement capables de gonfler à la chaleur à une température supérieure ou égale à 45 °C, en particulier supérieure ou égale à 50 °C, plus particulièrement supérieure ou égale à 60 °C, voire supérieure ou égale à 70 °C.

Comme indiqué précédemment la présente invention concerne également un procédé de maquillage, utilisant une composition cosmétique comprenant au moins des particules capables de s'expanser sous l'action d'une température supérieure ou égale à 45 °C, notamment une composition telle que définie précédemment. Ce procédé comprend l'application de la composition sur le support à traiter. Il comprend en outre le chauffage de la composition en particulier pendant ou postérieurement à son application.

Le chauffage de la composition est effectué dans des conditions qui permettent l'augmentation du volume de ladite composition par rapport au volume qu'elle occupe préalablement à son chauffage.

Le support à traiter peut être la peau, les lèvres, et les fibres kératiniques. Par « fibres kératiniques », on entend les cils et les sourcils.

Le support à traiter peut également être constitué d'un accessoire de maquillage comme des faux cils, postiches ou encore des pastilles ou des patchs adhérents sur la peau ou les lèvres (de type mouches).

Dans le procédé de l'invention, la composition est généralement chauffée à une température supérieure ou égale à 45 °C, notamment supérieure ou égale à 50 °C, en particulier supérieure ou égale à 60 °C et plus particulièrement supérieure ou égale à 70 °C. Evidemment, la température de chauffage dépend en particulier de la température susceptible d'être supportée par le support traité.

Selon un premier mode de réalisation du procédé selon l'invention, la composition est chauffée lors de son application. Dans un tel cas, le moyen de chauffage utilisé est généralement l'applicateur lui-même. Ainsi, dans le cas d'un mascara, la composition peut être appliquée à l'aide d'une brosse chauffante.

Selon un second mode de réalisation, la composition est chauffée postérieurement à son application. Selon une première variante, la composition peut être chauffée à l'aide de moyens non spécifiquement destinés à un chauffage, comme par exemple un corps occasionnellement chaud telle qu'une tasse ou une boisson chaude. Selon une seconde variante de ce mode de réalisation, la composition peut être chauffée à l'aide d'un moyen spécifiquement dédié au chauffage. Il peut en particulier s'agir d'un moyen propulsant de l'air chaud tel qu'un sèche-cheveux ou un dispositif de chauffage par exemple tel que décrit ci-après.

La composition selon l'invention peut être conditionnée dans un ensemble de conditionnement et d'application comprenant :
i) un réservoir comprenant une composition comprenant au moins des particules capables de s'expanser sous l'action d'une température supérieure ou égale à 45 °C, notamment telle que définie précédemment,
ii) un dispositif pour l'application de la composition, et
iii) des moyens de chauffage.

Selon un mode de réalisation, les moyens de chauffage sont formés par un dispositif distinct du dispositif ou organe d'application, l'ensemble étant configuré sous forme d'un dispositif de conditionnement et d'application comprenant en outre un récipient contenant une composition conforme à l'invention. Un tel dispositif peut être conditionné à l'intérieur d'un conditionnement du type blister pack. Les moyens de chauffage peuvent être du type de ceux décrits dans les brevets US 6 009 884 ou US 5 853 010. D'autres dispositifs configurés sous forme d'une pince chauffante (dans le cas des cils) peuvent également être utilisés. De tels dispositifs sont décrits notamment dans le brevet US 6 220 252.

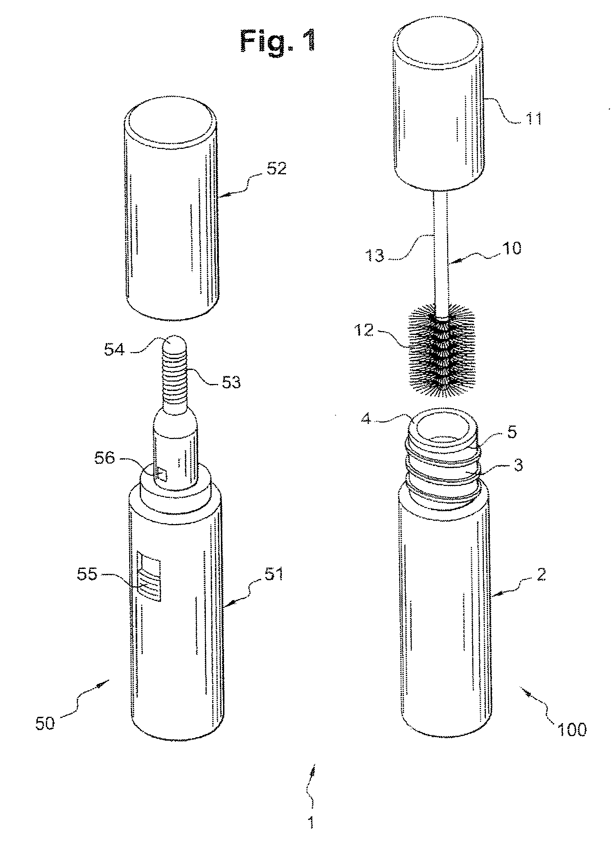

Le kit 1 décrit à la figure 1 comprend un ensemble de conditionnement et d'application 100 du mascara et un dispositif de chauffage 50, séparé de l'ensemble de conditionnement et d'application.

Les deux dispositifs 100 et 50 peuvent être vendus ensemble dans un même conditionnement, de type blister pack. L'unité 100 contenant le produit peut être vendue séparément.

L'ensemble de conditionnement et d'application 100 comprend un récipient 2, comprenant la composition selon l'invention, surmonté d'un col fileté 3 dont un bord libre délimite une ouverture 4. Dans l'ouverture 4, est monté un organe d'essorage 5. L'ensemble 100 comprend également un dispositif d'application 10 comprenant un bouchon 11 solidaire d'une tige 13 dont une extrémité comporte un applicateur 12, configuré généralement sous forme d'un arrangement de fibres maintenues entre les deux branches d'un fil de fer torsadé. Une surface intérieure du bouchon 11 est filetée de manière à coopérer avec le filetage du col 3. Ainsi, lorsque l'applicateur 12 et la tige 13 sont disposés à l'intérieur du récipient 2, le filetage du bouchon 11 vient en engagement avec le filetage du col 3 de manière à ce que le bouchon obture de manière étanche l'ouverture 4 du récipient. De tels ensembles de conditionnement et d'application sont bien connus.

Le dispositif de chauffage 50 est conforme à ce qui est décrit dans le brevet US 6 009 884. Il comprend principalement une partie de préhension 51 et un capuchon 52. Une batterie est disposée à l'intérieur de la partie de préhension 51, et est connectée à un fil chauffant 53 configuré sous forme d'un enroulement hélicoïdal disposé sur une tige 54. Un « switch » 55 permet de mettre en tension, respectivement d'éteindre le dispositif Une LED 56, lorsqu'elle change de couleur, indique que le dispositif est à la température requise, et qu'il est donc prêt à être utilisé.

L'alimentation de la partie chauffante via la batterie est en 12 V. La puissance dissipée est d'environ 1 Watt. Le fil chauffant 53 peut être réalisé en un alliage nickel/chrome.

Selon ce mode de réalisation, le mascara est appliqué à froid de manière classique sur les cils au moyen d'une brosse 12 puis chauffé après application : l'utilisatrice met en engagement la partie chauffante 53 du dispositif 50 avec les cils de manière à porter le dépôt de produit à la température d'expansion de la composition.

Les exemples figurant ci-après sont présentés à titre illustratif et non limitatif de l'invention.

### EXEMPLES

Dans les exemples ci-après, les teneurs indiquées sont exprimées en pourcentage en poids.

### Exemple 1 : Composition de mascara

| | |
|---|---|
| Particules non expansées commercialisées sous la dénomination d'« Expancel 007 WUF 40^{®} » par la société AKZO NOBEL | 10 |
| Hydroxyéthylcellulose | 1,71 |
| Talc | 4,5 |
| Propylène glycol | 6,48 |
| Oxyde de fer noir | 4,5 |
| Conservateur | 0,2 |
| Latex acrylique commercialisé sous la dénomination de « Syntran 5760^{®} » par la société Interpolymer à 40 % m.a. | 54 |
| Eau | qsp 100 |

On a étudié *in vitro* les caractéristiques de gonflement de cette composition de mascara.

Cette étude est effectuée sur une éprouvette de faux cils (cheveux caucasiens) sur laquelle on ne laisse que trois cils.

On applique la composition sur chacun des cils par dix passages. On mesure l'épaisseur après cinq minutes de séchage à température ambiante puis après chauffage des cils maquillés à 100 °C à l'aide d'un sèche-cheveux jusqu'à gonflement du dépôt.

On effectue trois types de mesure pour chacun des cils : (i) cil nu, (ii) cil maquillé et (iii) cil maquillé et chauffé, à l'aide d'une caméra « Navitar » en utilisant la loupe (x 2,5). Chaque mesure est effectuée en triple avec calcul de la moyenne obtenue.

Les résultats sont présentés dans le tableau 1 ci-après.

**TABLEAU 1**

| | Diamètre du cil nu (µm) | Diamètre du cil maquillé après séchage à température ambiante (µm) | Diamètre du cil maquillé et chauffé au sèche-cheveux 100 °C (µm) |
|---|---|---|---|
| Cil 1 | 92,1 ± 1 | 124 ± 0, 8 | 215 ± 6,2 |
| Cil 2 | 61,5 ± 0,7 | 111 ± 3 | 240 ± 4 |
| Cil 3 | 67,48 ± 0,8 | 108,2 ± 1,7 | 330 ± 27 |

On constate que la mise en oeuvre du procédé de maquillage décrit ci-dessus permet de doubler voire de multiplier par un facteur de 4,89 dans le cas du cil n° 3, le diamètre du cil.

On constate que la mise en oeuvre de la seule étape de chauffage permet de multiplier par un facteur d'au moins 3 l'épaisseur de la composition de mascara.

Le mascara de l'exemple 1 présente donc un effet volumateur très satisfaisant.

### Exemple 2 : Composition d'eye-liner

| | |
|---|---|
| Particules non expansées commercialisées sous la dénomination d'« Expancel 820 DU 40^{®} » par la société AKZO NOBEL | 5 |
| Dispersion acrylique commercialisées sous la dénomination de « Mexomère PAP^{®} » par la société CHIMEX à 24,5% en m.a | 71,4 |
| Mélange de copolymère tribloc butylène/éthylène/styrène et de copolymère étoile éthylène/propylène/styrène dans l'isododécane commercialisé sous la dénomination de « Versagel MD 960^{®} » par la société PENRECO | 1,3 |
| Octyl 2 dodécanol | 0,33 |
| Huile de parleam | 0,77 |
| Copolymère vinylpyrrolidone/1-eicosène commercialisé sous la dénomination d' « Antaron V220^{®} » par la société ISP | 0,44 |
| Phényl triméthicone | 0,77 |
| Oxyde de fer noir | 15 |
| Isododécane | qsp 100 |

### Exemple 3 : Composition de mascara de type émulsion

| | |
|---|---|
| Particules non expansées commercialisées sous la dénomination d'« Expancel 007 WU^{®} » par la société AKZO NOBEL | 10 |
| Cire (hydroxystéaroyloxy)stéarate d'alkyle en C₂₀-C₄₀ commercialisée sous la dénomination de « Kester wax K82 P^{®} » par la société KOSTER KEUNEN | 22,5 |
| Cire de candellila | 5,4 |
| Acide stéarique | 5,2 |
| Oxyde de fer noir | 5 |
| Aminométhyl propanediol | 0,45 |
| Isononanoate d'isononyle | 2,7 |
| Hydroxyéthylcellulose | 0,82 |
| Gomme arabique | 3,1 |
| Triéthanolamine | 2,16 |
| Conservateurs | 0,41 |
| Eau | qsp 100 |

### Exemple 4 : Composition de rouge à lèvres anhydre de type pâte souple (gloss)

| | |
|---|---|
| Polyacryladipate 2 de bis glycéryle | 16,14 |
| Malate de disostéaryle | 8,76 |
| Trimellilate de tridécyle | 9,31 |
| Triglycéride d'acide C₁₈-₃₆ | 17,69 |
| Silice diméthyl silylate | 8 |
| Pigments | 2,05 |
| Nacre | 3 |
| Polybutène | 12 |
| Tétraisostéarate de pentaérythrityle | 12,42 |
| Particules non expansées commercialisées sous la dénomination d'« Expancel 007 WU^{®} » par la Société AKZO NOBEL | 10 |
| Parfum, conservateurs | qs |

Ce rouge à lèvres est préparé par broyage des pigments dans une partie de la phase huileuse. Le reste de la phase huileuse est mis à chauffer dans un poêlon à double paroi au bain d'huile (90-95°) avec le polyisobutène. On y ajoute alors le broyat en maintenant le chauffage sous agitation avec un agitateur du type rayneri jusqu'à homogénéisation. On ajoute alors progressivement la nacre puis la silice en maintenant l'agitation et le chauffage à 90-95 °C jusqu'à obtention d'un mélange homogène.

Après refroidissement du mélange à température ambiante, on ajoute les particules d'« Expancel 007 WU^{®} » sous agitation au rayneri jusqu'à l'obtention d'une dispersion homogène des particules.

## Revendications

1. Composition cosmétique de maquillage choisie parmi un mascara, un eyeliner et un rouge à lèvres comprenant au moins des particules capables de s'expanser sous l'action d'une température supérieure ou égale à 45 °C.

2. Composition cosmétique selon la revendication 1, **caractérisée en ce qu'**elle est thermoexpansible.

3. Composition selon l'une quelconque des revendications 1 et 2, **caractérisée en ce que** lesdites particules sont capables de s'expanser sous l'action d'une température supérieure ou égale à 50 °C, en particulier à 60 °C, plus particulièrement à 70 °C.

4. Composition selon la revendication 3, **caractérisée en ce que** ladite température est supérieure ou égale à 80 °C, notamment à 85 °C, en particulier à 90 °C et plus particulièrement supérieure ou égale à 100 °C.

5. Composition selon l'une quelconque des revendications 1 à 4, **caractérisée en ce que** lesdites particules sont thermoplastiques.

6. Composition selon l'une quelconque des revendications 1 à 5, **caractérisée en ce que** lesdites particules sont des particules creuses comportant une enveloppe continue et au moins une cavité.

7. Composition selon la revendication 6, **caractérisée en ce que** ladite enveloppe comprend au moins un polymère résultant de la polymérisation de monomères choisis parmi les esters d'acides méthacrylique et acrylique, le chlorure de vinylidène, l'acrylonitrile, le styrène et ses dérivés, le butadiène et ses dérivés et leurs mélanges.

8. Composition selon la revendication 7, **caractérisée en ce que** ledit polymère est choisi parmi les polymères comportant au moins des motifs dérivés d'acrylate ou de méthacrylate de méthyle, les polymères comportant au moins des motifs dérivés d'acrylonitrile, les polymères comportant au moins des motifs dérivés d'acrylonitrile et de méthacrylate de méthyle, les polymères comportant au moins des motifs dérivés de styrène et d'acrylonitrile, les polymères comportant au moins des motifs dérivés de chlorure de vinylidène et d'acrylonitrile, les polymères comportant au moins des motifs dérivés de chlorure de vinylidène et de chlorure de vinyle.

9. Composition selon l'une quelconque des revendications 7 et 8, **caractérisée en ce que** ledit polymère est choisi parmi les polymères chlorure de vinylidène/acrylonitrile/méthacrylate de méthyle, les polymères acrylonitrile/méthacrylate de méthyle et les homopolymères d'acrylonitrile.

10. Composition selon l'une quelconque des revendications 6 à 9, **caractérisée en ce que** ladite cavité contient au moins un composé capable de manifester en réponse à un chauffage à une température située dans une gamme allant de 45 °C à 200 °C, une augmentation d'au moins un facteur 30 de son volume par rapport à son volume à température ambiante.

11. Composition selon la revendication 10, **caractérisée en ce que** ledit composé est un gaz choisi parmi l'air, l'azote, les hydrocarbures gazeux et leurs mélanges.

12. Composition selon la revendication 10 ou 11, **caractérisée en ce que** ledit composé est un composé liquide présentant une température de vaporisation allant de 45 °C à 200 °C, en particulier de 80 °C à 200 °C.

13. Composition selon la revendication 12, **caractérisée en ce que** ledit composé liquide est choisi parmi les hydrocarbures ayant de 5 à 15 atomes de carbone.

14. Composition selon l'une quelconque des revendications 1 à 13, **caractérisée en ce que** lesdites particules sont globulaires ou allongées.

15. Composition selon la revendication 14, **caractérisée en ce que** lesdites particules sont sphériques et présentent une granulométrie, exprimée en diamètre effectif moyen en poids, allant de 0,5 µm à 200 µm, notamment de 1 µm à 100 µm, en particulier de 4 µm à 50 µm et plus particulièrement de 5 µm à 40 µm.

16. Composition selon l'une quelconque des revendications 1 à 14, **caractérisée en ce que** lesdites particules sont des fibres, ayant en particulier une longueur allant de 0,05 mm à 6 mm.

17. Composition selon l'une quelconque des revendications 1 à 16, **caractérisée en ce que** lesdites particules présentent une masse volumique allant de 500 kg/m³ à 5000 kg/m³, en particulier de 900 kg/m³ à 3000 kg/m³, et plus particulièrement de 900 kg/m³ à 2000 kg/m³.

18. Composition selon l'une quelconque des revendications 1 à 17, **caractérisée en ce que** lesdites particules sont présentes en une teneur allant de 0,05 % à 50 %, en particulier de 0,1 % à 40 %, plus particulièrement de 0,5 % à 30 % en poids par rapport au poids total de la composition.

19. Composition selon l'une quelconque des revendications précédentes, **caractérisée en ce qu'**elle comprend en outre un milieu cosmétiquement acceptable.

20. Composition cosmétique selon l'une quelconque des revendications précédentes, **caractérisée en ce qu'**elle est capable de gonfler à la chaleur à une température supérieure ou égale à 45 °C, en particulier supérieure ou égale à 50 °C, plus particulièrement supérieure ou égale à 60 °C, voire supérieure ou égale à 70 °C.

21. Composition selon l'une quelconque des revendications précédentes, **caractérisée en ce qu'**elle comprend en outre au moins un ingrédient supplémentaire cosmétiquement acceptable choisi parmi : des polymères filmogènes, des matières colorantes, des charges, des épaississants ou gélifiants, des agents tensioactifs, des cires, des plastifiants, des antioxydants, des conservateurs, des parfums, des neutralisants et des actifs cosmétiques.

22. Composition selon l'une quelconque des revendications 1 à 20, **caractérisée en ce qu'**elle comprend en outre au moins une matière colorante.

23. Composition selon l'une quelconque des revendications 1 à 22, **caractérisée en ce qu'**il s'agit d'une composition cosmétique de maquillage des fibres kératiniques.

24. Procédé de maquillage **caractérisé en ce qu'**il comprend le fait d'appliquer sur le support à traiter une composition telle que définie dans l'une quelconque des revendications précédentes et **en ce qu'**il comprend en outre le fait de chauffer ladite composition, en particulier pendant ou postérieurement à ladite application, dans des conditions permettant l'augmentation du volume de ladite composition par rapport à son volume non chauffé.

25. Support maquillé constitué d'un accessoire de maquillage comprenant un maquillage susceptible d'être obtenu selon le procédé tel que défini selon la revendication précédente.

26. Ensemble de conditionnement et d'application (100) comprenant :
i) un réservoir (2) comprenant une composition comprenant au moins des particules capables de s'expanser sous l'action d'une température supérieure ou égale à 45 °C,
ii) un dispositif (10) pour l'application de la composition, et
iii) des moyens de chauffage (50).

27. Ensemble de conditionnement et d'application selon la revendication 26, dans lequel le réservoir comprend une composition selon l'une quelconque des revendications 1 à 23.

28. Utilisation de particules thermoexpansibles telles que définies selon l'une quelconque des revendications 1 à 18 à titre d'additif pour conférer un effet volumateur sous l'effet de la chaleur dans une composition cosmétique de maquillage choisie parmi un mascara, un eyeliner et un rouge à lèvres.

## Patentansprüche

1. Kosmetische Schminkzusammensetzung, die gewählt ist aus einer Wimperntusche, einem Eyeliner und einem Lippenstift, die wenigstens Partikel enthält, die sich unter der Einwirkung einer Temperatur größer oder gleich 45 °C ausdehnen können.

2. Kosmetische Zusammensetzung nach Anspruch 1, **dadurch gekennzeichnet, dass** sie wärmeausdehnbar ist.

3. Zusammensetzung nach einem der Ansprüche 1 und 2, **dadurch gekennzeichnet, dass** sich die Partikel unter der Wirkung einer Temperatur größer oder gleich 50 °C, genauer größer oder gleich 60 °C und noch genauer größer oder gleich 70 °C ausdehnen können.

4. Zusammensetzung nach Anspruch 3, **dadurch gekennzeichnet, dass** die Temperatur größer oder gleich 80 °C, genauer größer oder gleich 85 °C, noch genauer größer oder gleich 90 °C und insbesondere größer oder gleich 100 °C ist.

5. Zusammensetzung nach einem der Ansprüche 1 bis 4, **dadurch gekennzeichnet, dass** die Partikel thermoplastische Partikel sind.

6. Zusammensetzung nach einem der Ansprüche 1 bis 5, **dadurch gekennzeichnet, dass** die Partikel hohle Partikel sind, die eine ununterbrochene Hülle und wenigstens einen Hohlraum aufweisen.

7. Zusammensetzung nach Anspruch 6, **dadurch gekennzeichnet, dass** die Hülle wenigstens ein Polymer enthält, das sich aus der Polymerisation von Monomeren ergibt, die aus Metacryl- und Acrylsäureestern, Vinylidenchlorid, Acrylonitril, Styrol und seinen Derivaten, Butadien und seinen Derivaten und ihren Gemischen gewählt sind.

8. Zusammensetzung nach Anspruch 7, **dadurch gekennzeichnet, dass** das Polymer gewählt ist aus den Polymeren, die wenigstens Einheiten enthalten, die von Acrylat oder Methyl-Metacrylat abgeleitet sind, den Polymeren, die wenigstens Einheiten enthalten, die von Acrylonitril abgeleitet sind, den Polymeren, die wenigstens Einheiten enthalten, die von Acrylonitril und Methyl-Metacrylat abgeleitet sind, den Polymeren, die wenigstens Einheiten enthalten, die von Styrol und Acrylonitril abgeleitet sind, den Polymeren, die wenigstens Einheiten enthalten, die von Vinylidenchlorid und Acrylonitril abgeleitet sind, und den Polymeren, die wenigstens Einheiten enthalten, die von Vinylidenchlorid und Vinylchlorid abgeleitet sind.

9. Zusammensetzung nach einem der Ansprüche 7 und 8, **dadurch gekennzeichnet, dass** das Polymer gewählt ist aus den Vinylidenchlorid-/Acrylonitril-/Methyl-Metacrylat-Polymeren und den Acrylonitril-/Methyl-Metacrylat-Polymeren und den Acrylonitril-Homopolymeren.

10. Zusammensetzung nach einem der Ansprüche 6 bis 9, **dadurch gekennzeichnet, dass** der Hohlraum wenigstens eine Verbindung enthält, die in Reaktion auf eine Erwärmung auf eine Temperatur, die in einem Bereich von 45 °C bis 200 °C liegt, eine Vergrößerung ihres Volumens in Bezug auf ihr Volumen bei Umgebungstemperatur um wenigstens einen Faktor 30 zeigen kann.

11. Zusammensetzung nach Anspruch 10, **dadurch gekennzeichnet, dass** die Verbindung ein Gas ist, das gewählt ist aus Luft, Stickstoff, gasförmigen Kohlenstoffwasserstoffen und ihren Gemischen.

12. Zusammensetzung nach Anspruch 10 oder 11, **dadurch gekennzeichnet, dass** die Verbindung eine flüssige Verbindung ist, die eine Verdampfungstemperatur im Bereich von 45 °C bis 200 °C, genauer von 80 °C bis 200 °C, zeigt.

13. Zusammensetzung nach Anspruch 12, **dadurch gekennzeichnet, dass** die flüssige Verbindung gewählt ist aus Kohlenstoffwasserstoffen, die 5 bis 15 Kohlenstoffatome haben.

14. Zusammensetzung nach einem der Ansprüche 1 bis 13, **dadurch gekennzeichnet, dass** die Partikel kugelförmig oder langgestreckt sind.

15. Zusammensetzung nach Anspruch 14, **dadurch gekennzeichnet, dass** die Partikel sphärisch sind und eine Granulometrie, ausgedrückt als mittlerer effektiver Durchmesser nach Masseteilen, im Bereich von 0,5 um bis 200 µm, genauer von 1 µm bis 100 µm, noch genauer von 4 µm bis 50 µm und insbesondere von 5 µm bis 40 µm, zeigen.

16. Zusammensetzung nach einem der Ansprüche 1 bis 14, **dadurch gekennzeichnet, dass** die Partikel Fasern sind, die insbesondere eine Länge im Bereich von 0,05 mm bis 6 mm haben.

17. Zusammensetzung nach einem der Ansprüche 1 bis 16, **dadurch gekennzeichnet, dass** die Partikel eine Dichte im Bereich von 500 kg/m³ bis 5000 kg/m³, genauer von 900 kg/m³ bis 3000 kg/m³ und noch genauer von 900 kg/m³ bis 2000 kg/m³ haben.

18. Zusammensetzung nach einem der Ansprüche 1 bis 17, **dadurch gekennzeichnet, dass** die Partikel einen Anteil im Bereich von 0,05 Gew.-% bis 50 Gew.-%, genauer von 0,1 Gew.-% bis 40 Gew.-% und noch genauer von 0,5 Gew.-% bis 30 Gew.-% in Bezug auf das Gesamtgewicht der Zusammensetzung aufweisen.

19. Zusammensetzung nach einem der vorhergehenden Ansprüche, **dadurch gekennzeichnet, dass** sie außerdem ein kosmetisch akzeptables Milieu enthält.

20. Kosmetische Zusammensetzung nach einem der vorhergehenden Ansprüche, **dadurch gekennzeichnet, dass** sie bei Wärme mit einer Temperatur größer oder gleich 45 °C, genauer größer oder gleich 50 °C, noch genauer größer oder gleich 60 °C und insbesondere größer oder gleich 70 °C quellen kann.

21. Zusammensetzung nach einem der vorhergehenden Anspruche, **dadurch gekennzeichnet, dass** sie außerdem wenigstens einen zusätzlichen kosmetisch akzeptablen Inhaltsstoff enthält, der gewählt ist aus: filmogenen Polymeren, Farbstoffen, Ladungen, Verdickungsmitteln oder Geliermitteln, grenzflächenaktiven Stoffen, Wachsen, Weichmachern, Antioxidanzien, Konservierungsmitteln, Parfums, Neutralisatoren und aktiven kosmetischen Stoffen.

22. Zusammensetzung nach einem der Ansprüche 1 bis 20, **dadurch gekennzeichnet, dass** sie außerdem wenigstens einen Farbstoff enthält.

23. Zusammensetzung nach einem der Ansprüche 1 bis 22, **dadurch gekennzeichnet, dass** es sich um eine kosmetische Schminkzusammensetzung aus keratinartigen Fasern handelt.

24. Schminkverfahren, **dadurch gekennzeichnet, dass** es den Schritt umfasst, auf den zu behandelnden Träger eine Zusammensetzung aufzubringen, wie sie in einem der vorhergehenden Ansprüche definiert ist, und dass es außerdem den Schritt umfasst, die Zusammensetzung insbesondere während oder nach der Aufbringung unter Bedingungen zu erwärmen, die die Erhöhung des Volumens der Zusammensetzung in Bezug auf ihr nicht erwärmtes Volumen ermöglichen.

25. Geschminkter Träger, der aus einem Schmink-Accessoire gebildet ist, das eine Schminke enthält, die gemäß dem Verfahren nach dem vorhergehenden Anspruch erhalten werden kann.

26. Verpackungs- und Anwendungsanordnung (100), die umfasst:
i) einen Vorratsbehälter (2), der eine Zusammensetzung enthält, die wenigstens Partikel umfasst, die sich unter der Einwirkung einer Temperatur größer oder gleich 45 °C ausdehnen können,
ii) eine Vorrichtung (10) zum Anwenden der Zusammensetzung und
iii) Heizmittel (50).

27. Verpackungs- und Anwendungsanordnung nach Anspruch 26, bei der der Vorratsbehälter eine Zusammensetzung nach einem der Ansprüche 1 bis 23 enthält.

28. Verwendung von wärmeausdehnbaren Partikeln, wie sie in einem der Ansprüche 1 bis 18 definiert sind, als Zusatzstoff, um unter der Wirkung von Wärme in einer kosmetischen Schminkzusammensetzung, die aus einer Wimperntusche, einen Eyeliner und einem Lippenstift gewählt ist, eine Volumenausdehnungswirkung zu erzielen.

## Claims

1. A makeup cosmetic composition chosen from a mascara, an eyeliner and a lipstick comprising at least some particles that are capable of expanding under the action of a temperature of greater than or equal to 45°C.

2. A cosmetic composition according to claim 1, **characterized in that** it is heat-expandable.

3. Composition according to claim 1 or claim 2, **characterized in that** the said particles are capable of expanding under the action of a temperature of greater than or equal to 50°C, in particular 60°C and more particularly 70°C.

4. A composition according to claim 3, **characterized in that** the said temperature is greater than or equal to 80°C, especially 85°C, in particular 90°C and more particularly greater than or equal to 100°C.

5. A composition according to any one of claims 1 to 4, **characterized in that** the said particles are thermoplastic.

6. A composition according to any one of claims 1 to 5, **characterized in that** the said particles are hollow particles comprising a continuous envelope and at least one cavity.

7. A composition according to claim 6, **characterized in that** the said envelope comprises at least one polymer resulting from the polymerization of monomers chosen from methacrylic and acrylic acid esters, vinylidene chloride, acrylonitrile, styrene and derivatives thereof, butadiene and derivatives thereof, and mixtures thereof.

8. A composition according to claim 7, **characterized in that** the said polymer is chosen from polymers comprising at least some methyl acrylate or methacrylate derivatives, polymers comprising at least some acrylonitrile derivatives, polymers comprising at least some acrylonitrile and methyl methacrylate derivatives, polymers comprising at least some styrene and acrylonitrile derivatives, polymers comprising at least some vinylidene chloride and acrylonitrile derivatives, and polymers comprising at least some vinylidene chloride and vinyl chloride derivatives.

9. A composition according to claim 7 or claim 8, **characterized in that** the said polymer is chosen from vinylidene chloride/acrylonitrile/methyl methacrylate polymers, acrylonitrile/methyl methacrylate polymers and acrylonitrile homopolymers.

10. A composition according to any one of claims 6 to 9, **characterized in that** the said cavity contains at least one compound capable of exhibiting, in response to heating to a temperature within the range from 45°C to 200°C, an increase by at least a factor of 30 of its volume relative to its volume at room temperature.

11. A composition according to claim 10, **characterized in that** the said compound is a gas chosen from air, nitrogen and gaseous hydrocarbons, and mixtures thereof.

12. A composition according to claim 10 or claim 11, **characterized in that** the said compound is a liquid compound with a vaporization temperature ranging from 45°C to 200°C and in particular from 80°C to 200°C.

13. A composition according to claim 12, **characterized in that** the said liquid compound is chosen from hydrocarbons containing from 5 to 15 carbon atoms.

14. A composition according to any one of claims 1 to 13, **characterized in that** the said particles are globular or elongated.

15. A composition according to claim 14, **characterized in that** the said particles are spherical and have a particle size, expressed as the weight-average effective diameter, ranging from 0.5 µm to 200, µm, especially from 1 µm to 100 µm, in particular from 4 µm to 50 µm and more particularly from 5 µm to 40 µm.

16. A composition according to any one of claim 1 to 14, **characterized in that** the said particles are fibres, in particular having a length ranging from 0.05 mm to 6 mm.

17. A composition according to any one of claims 1 to 16, **characterized in that** the said particles have a mass per unit volume ranging from 500 kg/m³ to 5000 kg/m³, in particular from 900 kg/m³ to 3000 kg/m³ and more particularly from 900 kg/m³ to 2000 kg/m³.

18. A composition according to any one of claims 1 to 17, **characterized in that** the said particles are present in a content ranging from 0.05% to 50%, in particular from 0.1% to 40% and more particularly from 0.5% to 30% by weight relative to the total weight of the composition.

19. A composition according to any one of the preceding claims, **characterized in that** it also comprises a cosmetically acceptable medium.

20. A cosmetic composition according to any one of the preceding claims, **characterized in that** it is capable of swelling on heating at a temperature of greater than or equal to 45°C, in particular greater than or equal to 50°C and more particularly greater than or equal to 60°C., or even greater than or equal to 70°C.

21. A composition according to any one of the preceding claims, **characterized in that** it also comprises at least one additional cosmetically acceptable ingredient chosen from:
film-forming polymers, dyestuffs, fillers, thickeners or
gelling agents, surfactants, waxes, plasticizers, antioxidants, preserving agents, fragrances, neutralizers and
cosmetic active agents.

22. A composition according to any one of claim 1 to 20, **characterized in that** it also comprises at least one dyestuff.

23. A composition according to any one of claims 1 to 22, **characterized in that** it is a makeup cosmetic composition for keratin fibres.

24. A makeup process **characterized in that** it comprises the application to the support to be treated of a composition as defined in any one of the preceding claims and **in that** it further comprises heating the said composition, in particular during or after the said application, under conditions allowing an increase in the volume of the said composition relative to its unheated volume.

25. A made-up support constituted of a makeup accessory comprising a makeup that may be obtained according to the process as defined according to the preceding claim.

26. A packaging and application assembly (100) comprising:
i)a reservoir (2) comprising a composition comprising at least some particles capable of expanding under the action of a temperature of greater than or equal to 45°C;
ii) a device (10) for applying the composition; and
iii) heating means (50).

27. A packaging and application assembly according to claim 26, wherein the reservoir comprises a composition according to any one of claims 1 to 23.

28. A use of heat-expandable particles as defined according to any one of claims 1 to 18 as additive for giving a volumizing effect under the effect of heat in a cosmetic composition of makeup chosen from a mascara, an eyeliner and a lipstick.
